# EUROPEAN PATENT APPLICATION

(11) **EP 0 592 870 A1**
(43) Date of publication of application: **20.04.1994**
(21) Application number: 93115730.9
(22) Date of filing: 29.09.1993
(51) Int. Cl.: A61L 29/00, A61L 33/00

(54) **Process for preparing functionally coated expanded products from expandable tubing and the expanded products produced thereby**

(30) Priority: 30.09.1992 US 954906
(71) Applicant: C.R. BARD, INC., Murray Hill New Jersey 07974 (US)
(72) Inventor: Tedeschi, Eugene, Still River, Mass. (US); Elton, Richard K., Glens Falls, N.Y. (US)
(74) Representative: Laufhütte, Dieter, Dr.-Ing.

(57) **Abstract**

A process for preparing expanded thermoplastic medical products (e.g., dilatation balloons) having biomedical functional coatings such as lubricous coatings or protective coatings is disclosed. The process involves the steps of coating a suitable expandable thermoplastic tubing with the biomedical functional coating, at least partially drying the coated tubing, and then stretching and expanding the coated tubing. The coating comprises at least one functional agent (e.g., poly(ethylene oxide), a hydrophilic polyurethane or polysiloxane) dispersed, or emulsified in a carrier such as water and/or an organic solvent. Conventional fabricating procedures are used for stretching and expanding the coated tubing. Optional steps may include a heating step during stretching and expansion. The coated products produced by this process have an uniformly functional coating.

## Description

### BACKGROUND OF THE INVENTION

This invention relates to expanded thermoplastic medical products, particularly to a novel process for preparing an expanded thermoplastic medical product having a functional biomedical coating adhered to the expanded surface, particularly coated balloons for use on catheters.

The prior art process for applying functional coatings to balloons utilizes the step of applying the coating to the pre-formed balloon, i.e., to the blow molded balloon. Preferably the coating is applied to the inflated pre-formed balloon. Applying the coating to the inflated balloon allows the formation of a coating layer having a generally uniform thickness. A typical coating procedure is described at Col. 4, lines 48-54 and exemplified at Col. 5, lines 41-44 of U.S. 5,026,607 (issued June 25, 1991 to M. Kiezulas). The dilatation balloons are first formed from the appropriate tubing by a series of stretching, optionally heating, and expanding operations such as blow molding. Then the balloon is bonded to an appropriate catheter shaft using an adhesive or other appropriate means. Alternatively the tubing may be an integral part of the catheter. Finally, the functional coatings are applied, typically by dipping or spraying. The coated surface is allowed to dry and cured if necessary.

Current functional coatings used on pre-formed balloons are generally limited to water borne dispersions and emulsions because most organic solvents tend to permeate the thin wall of the balloon and alter the physical properties, e.g., by changing the polymer crystallinity.

There are several disadvantages to coating processes which involve applying the functional coating to a pre-formed balloon. These disadvantages include the following:
(i) the coating thicknesses on irregularly shaped objects can be inconsistent due to non-uniform drainage of the coating solution as it flows off the coated object, and some balloon geometries may promote entrapment of air during coating, thus producing a product with cosmetic irregularities;
(ii) pre-formed balloons are highly oriented and crystalline and thus are very sensitive to changes caused by contact with the solvents present in some coating solutions; and are also sensitive to any heating operations that may be required for curing a coating or driving off solvent from the coating solution;
(iii) to minimize the effect of solvent exposure on pre-formed balloons it may be necessary to apply the coating by spraying rather than dipping, in which case there are safety concerns with flammability, exposure to the finely divided solvent mist, and unwanted side effects on the coating due to evaporative cooling of the finely divided droplets formed during spraying; and
(iv) the high value loss of coated pre-formed balloons and coated finished catheters which are caused by coating errors.

### SUMMARY OF THE INVENTION

It is an object of the invention to coat an expandable thermoplastic tubing material suitable for a medical product with a coating containing a functional biomedical material and a carrier and to then expand the coated tubing to form an expanded medical product with an adherent coating of the functional biomedical material.

It is also an object of this invention to coat an expandable thermoplastic tubing suitable for forming a balloon with a coating which contains a functional biomedical material and to expand the coated tubing to form an expanded balloon with a uniformly functional, highly adherent coating on its outer surface.

It is a further object of the invention to coat an expandable thermoplastic tubing suitable for blow molding a dilatation balloon with a lubricious coating and to blow mold the coated tubing to form a lubricious dilatation balloon.

The present invention provides a process for preparing a coated expanded thermoplastic medical product. The process comprises the steps of:
(a) applying to at least a portion of an inner and/or an outer surface of an expandable thermoplastic tubing at least one adherent functional biomedical coating which comprises at least one functional agent and a carrier;
(b) allowing the coated tubing to dry at a time and temperature sufficient to remove at least part of the carrier; and
(c) stretching and/or expanding the dried coated tubing with an inflating fluid at a pressure sufficient to form the functionally coated expanded medical product. The expanded thermoplastic medical products prepared by this process are uniformly functionally coated.

The present invention provides a process for preparing a coated balloon. The process comprises the steps of:
(a) applying to at least a portion of an inner and/or an outer surface of an expandable thermoplastic tubing at least one adherent functional biomedical coating which comprises at least one functional agent and a carrier;
(b) allowing the coated tubing to dry at a time and temperature sufficient to remove at least part of the carrier; and
(c) stretching and/or expanding the dried coated tubing with an inflating fluid at a pressure sufficient to form the functionally coated balloon. The balloons prepared by this process are uniformly functionally coated.

The present invention also provides a process for preparing a lubricious dilatation balloon. The process comprises the steps of:
(a) applying to at least a portion of an outer surface of an expandable thermoplastic tubing at least one adherent functional biomedical coating which comprises (i) one or more carriers, (ii) a poly(ethylene oxide) dissolved, dispersed, or emulsified in the carrier, and (iii) a polymer selected from the group consisting of a polyurethane, a polyurea, and a polyurethane-urea or polymer precursors selected from the group consisting of polyurethane precursors and polyurea precursors, the polymer or polymer precursors being dissolved, dispersed, or emulsified in the carrier(s);
(b) allowing the coated tubing to dry at a time and temperature sufficient to remove at least part of the carrier; and
(c) expanding the coated tubing with an inflating fluid in order to increase the diameter and length of the tubing while simultaneously orienting the tubing. The lubricious dilatation balloons prepared by this process are uniformly functionally coated.

As used herein, the term "thermoplastic" is exclusive of natural or synthetic rubbers. It is understood that thermoplastic materials which can be formed into a tube, coated, and then subjected to further heat treatment to cure the coating and/or the tubing and form a thermoset tube are also suitable for use herein.

As used herein, the term "functional biomedical coating" is used for coatings applied to medical products intended for use in the body. Such coatings are usually, but not necessarily activated in an aqueous environment, and they provide (i) protective properties such as abrasion resistance, puncture resistance and/or tear resistance, (ii) functional properties such as lubricity, thromoboresistance, hemocompatibility, and (iii) therapeutic properties such as anti-thrombogenic properties, antimicrobial properties and the like, and like functional improvements. The functional biomedical coatings useful herein must exhibit adhesion suitable for the intended end use of the medical product and they should have the potential for reasonably high radial and circumferential stretching.

The carriers used for the coatings can be either an organic solvent or water, with the choice depending on the coating components and on the thermoplastic tubing material being coated. In some cases one or more organic solvents may be used, whereas in other cases water may be used as the carrier. Suitable solvents are those which do not adversely affect stretching and expanding steps which are used to form the final expanded product from the coated tubing. Suitable solvents include alcohols such as ethanol or ethylene glycol; ketones such as methyl ethyl ketone; ethers such as ethylene glycol methyl ether; esters such as ethyl acetate; ether acetates such as ethylene glycol monoethyl ether acetate; aromatic and aliphatic solvents such as benzene, toluene, xylene, N-methylpyrrolidone, and pyridine; nitroparaffins such as 2-nitropropane; chlorinated solvents such as methylene chloride, methylene bromide, trichloroethylene, ethylene dichloride; and mixtures of the above. The chlorined solvents are particularly useful for poly(ethylene oxide) containing coatings.

The ability to maintain a continuous coating over the surface of the thus-expanded balloon, even after the radial and circumferential stretching of several hundred percent which is involved in expanding the balloon, is highly advantageous.

Applying the functional biomedical coating to the expandable thermoplastic tubing prior to expansion provides the following advantages:
(i) it is possible to coat the tubing using solvents which would otherwise attack and deform a pre-formed polymeric material such as a dilatation balloon, thus greatly broadening the range of coatings which can be used;
(ii) it is much less expensive to discard rejected coated tubing than to discard rejected coated pre-formed objects such as catheter balloons or assembled catheters with attached balloons;
(iii) it is much easier to control the uniformity of the resulting coating because the material being coated, i.e., the tubing is a simple cylinder rather than a pre-formed object, such as a balloon, with a potentially complex geometry;
(iv) it is possible to readily cure coatings requiring elevated temperatures because the tubing, unlike pre-formed expanded products such as dilatation balloons or assembled products, can tolerate the significantly higher temperatures required to cure many coating compositions;
(v) separately coating the components of a multi-component device allows one to produce a device with different performance properties for each component, depending upon the coating used on each component, and this cannot be obtained if one is limited to coating the entire multi-component device; and
(vi) separately coating the individual components of a multi-component device lends itself to more efficient production because it allows simultaneous manufacturing steps to occur in parallel rather than in series.

### DESCRIPTION OF THE PREFERRED EMBODIMENTS

The expandable thermoplastic tubings to be coated include tubings made from the materials typically used to form expanded products such as dilatation balloons, esophageal dilatation balloons, fallopian tube dilatation balloons, peripheral angioplasty dilatation balloons, prostate dilatation balloons, coronary angioplasty balloons, uterine manipulator balloons and the like. Included among the suitable thermoplastic materials are polyurethanes, polyethylene, ethylene-butylene-styrene, polypropylene, polyethylene terephthalate or block copolymers thereof admixed with low molecular weight styrene and optionally polypropylene, and similar compositions employing butadiene or isoprene in place of the ethylene and butylene; polyvinyl chloride; copolyesters; silicone polycarbonate copolymers; ethylene-vinyl acetate copolymers; polyether polyamide block copolymers such as Pebax®; polycarbonates; ionomers such as Surlyn®; polybutylene terephthalate, polyarylether ketones; polyimides; polyamides, particularly aromatic polyamides; polyetherimides; polyamideimides; and the like. One skilled in the art will consider such factors as processability, performance parameters required for the balloon, coating adhesion, and biocompatibility in making the appropriate selection.

The choice of dilatation balloon material will depend on the particular application. For example, some applications will require a rigid non-compliant polymer, while other applications will favor the use of a softer polymer.

Segmented polyurethanes are suitable as thermoplastic tubing materials for dilatation balloons. Segmented polyurethanes can be varied by using different isocyanates and polyols and different ratios of hard to soft segments in the polymer. The hard to soft ratio defines the expansion capabilities of the balloon as well as its elasticity. If the segmented polyurethane to be coated is a hard polymer, then preferably a hard polymer should be used in the functional coating. If the segmented polyurethane to be coated is a soft polymer, then preferably a softer polymer should be used in the functional coating. Of particular interest for dilatation balloons are certain PELLETHANE® polyurethane polymers (e.g., PELLETHANE® 2367-75D).

Also particularly suitable as thermoplastic tubing materials for dilatation balloons are polyethylene terephthalates (PET), particularly high molecular weight homopolymers of PET and PET copolyesters. Balloons made from PET tend to be highly rigid when inflated and are capable of sustaining high inflation pressures even when the balloon wall thickness is thin, e.g., less than 0.002".

Similarly, there is a wide range of functional biomedical coatings which can be adapted for use in this process. The choice of coating will depend on factors such as the function desired (i.e., lubricity, hemocompatibility, antimicrobial properties, etc.), the ability of a particular coating to bond to the thermoplastic tubing material, the ability of the coating to endure the stretching and/or expanding process, durability (e.g. abrasion resistance) required of the coating in its intended application, and the like. One skilled in the art of coating will weigh these factors in making an appropriate choice.

Coatings containing poly(ethylene oxide) are desirable because poly(ethylene oxide) has the desired functional characteristic of "passivating" a surface toward platelets and it is quite non-thrombogenic. See Chapter 4 "Poly(ethylene oxide) and Related Hydrogels" by Neil B. Graham (pp. 95-113) in "Hydrogels in Medicine and Pharmacy", Vol. II Polymers, ed. by N. A. Peppas, CRC Press, Inc., Boca Raton, Florida, 1986.

Such coatings provide a combination of useful properties, namely, lubricity, biocompatibility and, when complexed with a complexing polymer, a high degree of durability. These properties are particularly useful and sought after in dilatation balloons.

Functional biomedical coatings containing poly(ethylene oxide) and pre-formed polyurethanes, polyureas, or polyurethaneureas are particularly useful, as are coatings containing poly(ethylene oxide) and polyurea precursors . Preferred coatings contain poly(ethylene oxide) blended with polyurethane precursors which are polymerized and crosslinked in situ on the coated surface.

Particularly preferred for applications requiring permanent lubricity, coating durability, abrasion resistance and a high poly(ethylene oxide) content are coatings of the type described in U.S. 5,077,352 (issued December 31, 1991 to R. K. Elton), the disclosure of which is incorporated herein by reference. These coatings are applied as a solution of an isocyanate, a polyol, and a high molecular weight poly(ethylene oxide).

After application of the coating, at least a portion of the solvent is removed and the coating is cured by allowing the polyol and isocyanate to react to form a crosslinked substantially polyurethane matrix associated or complexed with the poly(ethylene oxide) by hydrogen bonding. Preferably, the weight ratio of isocyanate and polyol to poly(ethylene oxide) varies from 0.25 to 6.0 and most preferably from 0.75 to 2.0. The stoichiometric ratio of total NCO groups in the isocyanate to total OH groups in the polyol may vary from 0.6 to 3.5, preferably from 0.85 to 1.5. Advantageously, when used as the coating in the present process, the Elton coating not only has the ability to adhesively bond by crosslinking but also the ability to soften, elongate, and conform to the thermoplastic tubing when moderate heat is applied to the coated tubing during the stretching and expanding of the coated tubing to form a balloon.

The particularly preferred coating of the Elton patent is prepared by weighing appropriate quantities of isocyanate, polyol, and poly(ethylene oxide), adding them to an appropriate solvent, and adding additional solvent as needed to adjust the solids content to the desired level. Suitable organic solvents for this coating may include acetonitrile, dioxolane, n-methyl pyrrolidone, pyridine, preferably halogenated solvents such as methylene chloride, methylene bromide, dichloroethane, dibromoethane, chloroform, and trichloroethylene, and blends of the above. Solids contents in a range of from 0.4 to 40% are preferred.

Another functional poly(ethylene oxide) coating useful herein is the hydrophilic coating described in U.S. 4,459,317 (issued July 10, 1984 to H.R. Lambert), the disclosure of which is incorporated herein by reference. A first coating of unreacted isocyanate groups on the polymer surface is formed by applying a solution containing a compound having at least two unreacted isocyanate groups per molecule and then evaporating the solvent by air drying. A second coating of poly(ethylene oxide) dissolved in a solvent is then applied, the solvent is evaporated off, and finally the coating is cured, preferably for 5-30 minutes at 50-100°C in the presence of a water-containing gas such as ambient air. Known isocyanate curing catalysts may be added to one or both coating solutions. The coating normally consists of a poly(ethylene oxide) - polyurea interpolymer formed when the isocyanate groups react with water during the curing to yield an amine which rapidly reacts with other isocyanate groups to form urea crosslinks.

Suitable isocyanates include polymethylene polyphenyl isocyanate, 4,4'-diphenylmethane diisocyanate, and 2,4-toluene diisocyanate, as well as prepolymers or other addition products of isocyanates and polyols. Suitable solvents for the isocyanate compound are ethyl acetate, acetone, chloroform, methyl ethyl ketone, ethylene dichloride, and preferably methylene chloride. The isocyanate solution may contain 0.5 to 10%, preferably 1 to 6% (weight/volume), of the isocyanate compound.

The poly(ethylene oxide) used should have a mean molecular weight of between 10⁴ and 10⁷, preferably 10⁵. A suitable poly(ethylene oxide) is sold under the trade name Polyox® by Union Carbide Corp. The preferred and suitable solvents for the poly(ethylene oxide) are the same as those discussed above. The proportion of poly(ethylene oxide) is preferably between 0.5 and 10%, most preferably between 2 and 8% (weight/volume).

Examples of other useful functional poly(ethylene oxide) coatings are found in U.S. 4,990,357 (issued Feb. 5, 1991 to M. Karakelle et al.). Of the several functional coatings disclosed in the '357 patent, the coatings containing poly(ethylene oxide) are useful herein for coating the thermoplastic tubing used to form dilatation balloons. Suitable coatings are uniform blends of an elastomeric segmented hydrophilic poly(urethanes) (HPEU) and poly(ethylene oxide) in an aqueous liquid. The poly(ethylene oxide) should be present in an amount of about 5 to 25 wt. %.

The HPEU is prepared from a diisocyanate, a polyether glycol, and a chain extender. Suitable diisocyanates are aromatic diisocyanates such as methylene diisocyanate (MDI), alicyclic diisocyanates such as isophorone diisocyanate and 4,4'-dicyclohexylmethane diisocyanate, and aliphatic diisocyanates, such as hexamethylene diisocyanate. The most preferred diisocyanate is MDI. The polyether glycol component may be polyethylene glycol (PEG), alone or mixed with polypropylene oxide glycol or polytetramethylene oxide glycol. The preferred polyol is PEG having a molecular weight of from about 600 to 3300 or a mixture containing at least 50 wt. % thereof. The most preferred polyether glycol is a PEG having an average molecular weight of 1000 to 1450. The chain extender may be water and/or a low molecular weight branched or unbranched diol, diamine or amino alcohol of up to 10 carbon atoms or mixtures thereof. The polymer thus formed is prepared without the use of a catalyst and the resulting product is a polyurethane or a polyurethaneurea depending upon the chain extender used.

Additional examples of useful functional poly(ethylene oxide) coatings are found in U.S. 5,041,100 (issued August 20, 1991 to S.M. Rowland et al.), the disclosure of which is incorporated herein by reference. Of the poly(ethylene oxide) coatings disclosed in the Rowland et al. patent, coatings where the structural plastic is a thermoplastic, solvent-soluble linear polyurethane are useful for coating the thermoplastic tubing used for forming dilatation balloons. These coatings comprise an intimate physical mixture of 50 to 98 wt. % of the polyurethane and 2 to 50 wt. % of a poly(ethylene oxide) having a molecular weight of at least 10,000, preferably at least 1,000,000. Coatings which contain 10% to 30% poly(ethylene oxide) are most useful herein. Typically, an aqueous dispersion of the polyurethane and poly(ethylene oxide) is used. There is no need for any curing step to effect cross-linking. The coatings provide at least temporary lubricity and may be beneficially employed in applications where temporary lubricity is sufficient or even desirable.

Other functional lubricious coatings useful herein include hydrophilic polyurethanes such as those disclosed in U.S. 4,156,066 and U.S. 4,156,067 (both issued May 22, 1979 to F.E. Gould); polyvinyl alcohols such as those disclosed in U.S. 4,977,901 (issued December 18, 1990 to R.F. Ofstead); hydrophilic polymers such as those described in U.S. 4,467,073 (issued August 21, 1984 to W.S. Creasey), U.S. 4,705,709 (issued November 10, 1987 to V.L. Vailancourt) and U.S. 4,876,126 (issued October 24, 1989 to N. Takemura et al.). The '066 hydrophilic polymers are polyurethane polyester resins having free hydroxyl groups and carboxylate groups in the polymer backbone. The functional protective lubricious coatings disclosed in U.S. 5,026,607 (issued June 25, 1991 to M. Kiezulas) are also useful herein. The '607 coatings comprise an aqueous dispersion of a urethane having a solids content of about 30-50% as the protective compound, a silicone or siloxane emulsion having a solids content of at least about 15% as a slip additive (e.g., dimethyl siloxane), and optionally a crosslinking agent for the urethane (e.g., a polyfunctional aziridine). The disclosure of the above patents is incorporated herein by reference.

Many functional lubricious coating used in biomedical applications contain a significant amount of polyvinyl pyrrolidinone (PVP). It is expected that such coatings will be useful herein provided the PVP is adequately modified to accept the conditions of the expansion step, for example, through the use of plasticizers, PVP copolymers, or other PVP modifiers.

For all functional biomedical coatings, it is understood that certain coating additives commonly used in formulating coatings may be beneficially employed to produce a desirable result. Exemplary coating additives include surfactants or wetting agents, viscosity and flow control agents, antioxidants, pigments, and air release agents or defoamers.

Surfactants or wetting agents are used to promote wetting to the substrate as well as adhesion of the coating to the substrate. Useful wetting agents include perfluoroalkyl ethoxylate mixtures, 2,4,7,9-tetramethyl 1-5-decyn-4,7-diol and ethylene oxide adducts thereof, 2,3,5-dimethyl-1, hexyn-3-ol, condensation products of ethylene oxide and di(isohexyl isoheptyl)phenol, condensation products of stearylamine and ethylene oxide, nonylphenoxypoly-(ethyleneoxy) ethanol, and polyethoxylated octylphenol.

Viscosity and flow control agents are used to adjust the viscosity and thixotropy to a desired level. Preferably the viscosity is such that the coating can be formed on the thermoplastic tubing at the desired thickness. Viscosities of from 50 cps to 500 cps can be used, although higher or lower viscosities may be useful in certain instances. Viscosity control agents include, but are not limited to, fumed silica, cellulose acetate butyrate and ethyl acrylate/2-ethylhexyl acrylate copolymer. Flow control agents are preferably used in amounts from 0.05 to 5 wt. % of coating.

Antioxidants are used to improve the oxidative stability of the coatings and include but are not limited, to tris (3,5-di-t-butyl-4-hydroxybenzyl)- isocyanurate, 2,2'-methylenebis(4-methyl-6-t-butyl phenol), 1,3,5-trimethyl-2,4,6-tris(3,5-di-butyl-4-hydroxybenzyl) benzene, butyl hydroxy toluene, octadecyl 3,5-di-t-butyl-4-hydroxyhydrocinnamate, 4,4'-methylenebis(2,6-di-t butylphenol), p,p'-dioctyl diphenylamine, 1,1,3-tris-(2-methyl-4-hydroxy-5-t-butylphenyl) butane. Antioxidants are preferably used in amounts from 0.01 to 1 wt. % of coating.

Conventional pigments can be added to impart color or radiopacity or to increase the desired appearance of the coatings.

Air release agents or defoamers are added to quickly release air bubbles formed in the wet film during the coating operation. They include, but are not limited, to polydimethyl siloxanes, 2,4,7,9-tetramethyl-5-decyn-4,7-diol, 2-ethylhexyl alcohol, and n-beta-aminoethyl-gamma-amino-propyl-trimethoxysilane. Air release agents are often used in amounts from 0.005 to 0.5 wt. % of the coating.

Additionally, the functional biomedical coatings may also contain an effective amount of a therapeutic agent which can diffuse out of the coating in a continuous, controlled dosage over a substantial period of time.

Specific examples of such therapeutic agents include anti-thrombogenic agents or other agents for suppressing acute thrombosis, stenosis or late restenosis in arteries. Such agents include heparin, streptokinase, urokinase, tissue plasminogen activator, anti-thromboxane B₂ agents, anti-B-thromboglobulin, prostaglandin E, aspirin, dipyridamole, anti-thromboxane A₂ agents, murine monoclonal antibody 7E3, triazolopyrimidine, ciprostene, hirudin, ticlopidine, nicorandil, and the like. Antiplatelet derived growth factor may be used as a therapeutic agent to suppress subintimal fibromuscular hyperplasia at an arterial stenosis site, or any other inhibitor of cell growth at the stenosis site may be used.

The therapeutic agent also may comprise a vasodilator to counteract vasospasm, for example, an antispasmodic agent such as papaverine. The therapeutic agent may be vasoactive agent such as calcium antagonists, or alpha and beta adrenergic agonists or antagonists.

The therapeutic agent may be an anti-neoplastic agent such as 5-fluorouracil or any known anti-neoplastic agent, optionally mixed with a controlled release carrier for the agent.

The therapeutic agent may be an antibiotic which may be applied, optionally in conjunction with a controlled release carrier for persistence, to an infected stent or any other source of localized infection within the body. Similarly, the therapeutic agent may comprise steroids for the purpose of suppressing inflammation in a localized tissue site.

Also, anti-infective agents such as chlorhexidine may be added for improved infection resistance of the coated product.

The coating may contain binders such as quaternary ammonium compounds and/or amino-functional poly(ethylene oxide) to modify the release kinetics of therapeutic agents carried in the coating.

Care should be taken in adding other agents to the coating mixture. The agents should be compatible with the functional agent and the agents used should not adversely affect each other's properties.

The above functional biomedical coatings are typically applied using conventional methods. One end of the tubing is sealed and the outer surface of the tubing is coated using a method such as dipping, spraying, wiping, painting or the like. Preferably, the tubing is dipped at about 20-25°C and less than 60% relative humidity. Coating thickness and functionality can be affected by withdrawal rate of the tubing from the coating mixture.

After applying the coating, the carrier is allowed to evaporate from the coated thermoplastic tubing, often by exposure to ambient conditions for a suitable drying time, typically from 15 to 480 minutes, or the tubing is dried at temperatures of 10 to 205°C from for from a few seconds to 48 hours. The time and temperature selected will, of course, depend upon the carrier used and the speed with which evaporation is desired. In all cases the drying conditions should be non-deleterious to the underlying thermoplastic tubing.

The coated thermoplastic tubing is fabricated into an expanded product using the conventional stretching and expanding steps used in blow molding. One skilled in the art will select the proper conditions, which will depend on the type of thermoplastic tubing coated and the type of functional coating applied. In most cases, the stretched tubing is heated during stretching and expansion.

When a coated dilatation balloon is being prepared by blow molding, the process described in U.S. Re. 32,983 (reissued July 11, 1989 to S.B. Levy) is typically used. The process described in the '983 patent is carried out by drawing at a suitable temperature a polymeric tubing having a finite length (L₁) and an internal diameter (ID₁) to a length (L₂) which is 3 to 6 L₁, then expanding the drawn tubing of internal diameter (ID₁) which is 6-8 ID, followed by cooling the drawn and expanded tubing to less than the second order transition temperature. The process can be carried out in conventional equipment. A mold having a cavity with dimensions commensurate with the desired size of the balloon to be produced is typically used. The open end of the tubing is equipped with a suitable fitting so that a pressurized fluid (e.g., a gas such as nitrogen) can be introduced into the tubing during the expansion step. If the tubing extends beyond the mold, the use of a restraining means is preferred to maintain the dimensions of the tubing in the region outside the mold while the pressure is being applied to the inside wall of the tubing. After the drawn tubing is positioned in the mold, heat is applied to raise the tubing's temperature. A suitable temperature is one within the range extending from the second order transition temperature to the first order transition temperature of the thermoplastic polymer used for the tubing. Similar temperatures can be used for both the drawing and expanding steps. The desired temperature can be achieved by any suitable heat generating means. For polyethylene terephthalate (PET) the preferred temperature is 84-99°C. For polyurethanes the temperature will vary depending upon the polyurethane's composition and the desired characteristic of the final product.

It will understood by one skilled in the art that some adjustments in the drawing and expansion ratios and in the drawing and expansion temperatures may be necessary to fabricate balloons. One skilled in the art will recognize that, although the tube drawing step is typically performed prior to the tube expansion step, the latter can be performed immediately after the drawing of the tubing, or it can be performed at a later time.

Because of the recovery characteristics of shaped polymeric structures which are drawn by the above procedures, it may be necessary to maintain axial tension on the drawn tubing during the expansion step.

The coated balloons are attached to the catheters using conventional methods such as crosslinking adhesives, hot melt adhesives, plastic welding, or the like. Typically, the catheters are not coated.

The present process can be used to coat the inflatable annular portion of integral dilatation catheters such as the combination guiding catheter-dilating catheter assembly described in U.S. 4,323,071 (issued April 6, 1982 to J.B. Simpson et al.). The assembly is formed of two heat-shrinkable irradiated modified polyolefin tubes, with the first tube being coaxially disposed within the second tube in such a manner that the second tube surrounds the first tube. Near the distal end of the second tube there is a balloon-like or inflatable annular portion. An annular flow passage is formed between the second tube and extends from the proximal end into the balloon-like or inflatable annular portion to permit the introduction of an expansion fluid into the balloon-like portion for inflating the balloon-like portion and for withdrawing the expansion fluid to deflate the balloon-like portion. The second tubular member is then progressively heated along its length in a suitable manner, such as by a hot air tool to a suitable temperature of about 120°. The second tube shrinks and forms a tight seal with respect to the first tube. Alternative methods for forming the catheter are disclosed in the '071 patent, the disclosure of which is incorporated herein by reference. Alternatively, the entire second tube of the combination can be coated instead of just the balloon. Any of the above functional biomedical coatings can be used and the balloon can then be inflated in the conventional manner.

It should be understood that the foregoing description of the invention is intended merely to be illustrative and that other embodiments and modifications may be apparent to those skilled in the art without departing from the spirit of the invention.

### Example 1

### a. Preparation of Premix

A poly(ethylene oxide) premix in methylene chloride was prepared by adding to about 3868 g. of methylene chloride about 132 g. of a poly(ethylene oxide) marketed under the trade name POLYOX® WSR N-750 NF resin. The POLYOX® WSR N-750 NF resin is a water-soluble resin (CAS Registry No. 25372-68-3) with an approximate molecular weight of 300,000. The mixture was rolled at 6-10 rpm for at least 24 hours. If the resulting solution was not uniform (i.e., if any lumps or gels were present), the lumps or gels were broken up and the solution was allowed to stand for 24 hours. The solution was then rolled for about 60 minutes to form a uniform solution of 3.3 wt. % total solids.

### b. Preparation of Coating

The final coating was prepared by adding a polyester polyol and an aromatic poly(isocyanate) to the poly(ethylene oxide) premix. The polyester polyol used was Multron R-18 which is available from the Coatings Division of Miles Corp. Multron R-18 is polyester resin having a hydroxyl number of 57-63, acid number of 1.2 maximum, water content of 0.10 wt. % maximum, Gardner color of No. 2 maximum, and viscosity at 73°C of 900-1600 mPa's. Typically, the colorless to light yellow liquid has a OH content of 1.8%, average equivalent weight of 935, specific gravity of 1.19 @ 5°C, weight per gallon of 9.9 lbs., viscosity @ 20°C of 26,000 mPa's, and flash point (Pensky-Marten closed cup) of 204°C (400°F). The isocyanate used was Mondur CB-60 which is available from the Coatings Division of Miles Corp. Mondur CB-60 is an aromatic polyisocyanate adduct based on toluene diisocyanate (TDI) dissolved in propylene glycol monomethyl ether acetate (PMA) and xylene (25:15). It has a solids content of 60 ± 2 wt. %, NCO content of 10.0 - 10.8%, Gardner color of No. 4 maximum, viscosity @ 20°C of 150-600 mPa's, and free TDI monomer content of 0.7% maximum. Typically, the clear, slightly yellow liquid has an equivalent average weight of 404, specific gravity @ 25°C of 1.13, weight of 9.4 lbs. per gallon, and flash point of 28°C (82°F).

A total of 6.72 g. of the polyester polyol was added to an appropriately sized container. Then 3.22 g. of the isocyanate were added, followed by 265.3 g. of the poly(ethylene oxide) premix and then by 421.7 g. of methylene chloride. The resulting mixture was mixed on a roller for at least 5 minutes and until it was visually homogenous. The coating solution had a total solids content of about 3 wt. %.

### c. Application of the Coating

Thermoplastic polyurethane tubings having inner diameters ranging from 0.0077 to 0.0238 in., wall thicknesses ranging from 0.0037 to 0.0119 in., and outer dimensions ranging from 0.0151 to 0.0476 were heat sealed at one end. The tubing used was PELLETHANE® 2363-75D available from Dow Chemical Co. PELLETHANE® 2363-75D is a polyether polyurethane resin used for extrusion and injection molding. Typically, it has a melt index of about 34 gm/10 min. (224°C, 5000 g.); Durometer D hardness of 74 ± 4; specific gravity of 1.21; tensile modulus of 165,000 psi; ultimate tensile strength of 6980 psi; ultimate elongation of 250%; tear strength (Die C) of 14 pli; heat distortation temperatures of 65 and 55 at 66 and 264 psi, respectively; Taber abrasion (H-22 wheel, 1000 g. weight, 1000 cycles) of 55 mg. loss; Clash-Berg Modulus (Tf) of +9°C; and flexural modulus of 190,000 psi. The ASTM test methods used are as follows: D-1238 for melt index; D-2240 for hardness; D-792 for specific gravity; D-412 for tensile modulus, ultimate tenule strength and ultimate elongation; D-624 for tear strength; D-648 for heat distortion temperature; D-1044 for Taber abrasion; D-1043 for Clash-Berg modulus, and D-790 for flexural modulus.

The coating was applied by dipping the tubing in the above solution. The tubing was introduced at a rate of about 25 inches per minute and withdrawn at a rate of about 15 inches/minute. The tubing was dried at room temperature for about 15-480 minutes and then cured at about 50°C for about 8 hours.

### d. Evaluation of the Coating on the Tubing

The surface of the tubing contained a continuous coating of a crosslinked polyurethane complexed or associated with poly(ethylene oxide). When viewed under an optical microscope, the smooth coating was observed to be free from fissuring, crazing or other signs of coating rupture.

Coating continuity was evaluated using a 0.5% aqueous solution of Congo Red. The solution was poured into an appropriately sized and shaped cylinder. The coated tubing to be tested was immersed in the solution for approximately 30 seconds. The tubing was then held under running tap water to remove excess Congo Red solution. There was a marked line of distinction where the coated portion of the tube met the uncoated section, as demonstrated by the red color. The tubing was then viewed under 20X magnification. A continuous red film was observed.

The wet coating on the tubing exhibited a high degree of lubricity as determined by rubbing the coated tubing between the fingers while applying a firm pressure, and comparing the ease of movement to that observed when performing the same test on a similar wet tubing which had not been coated. The uncoated tubing, even when wet, offered significantly more resistance to movement than the coated tubing.

The abrasion resistance of the coating was determined by placing the coated, stained tubing under a stream of running tap water again so that the coating would be rehydrated. While under the running water, the tubing was rubbed between the finger and thumb for a total of 20 times while applying firm finger pressure. After this, the coating still exhibited a continuous red color along the tubing, indicating that it did not wear off. Further examination for lubricity showed that the lubricity of the section of tubing given the above abrasion test was the same as a similar section that was not rubbed.

The integrity of the coating after the abrasion test was further verified by allowing the coated tubing to dry and reexamining the coated surface under a 50 X optical microscope. A continuous, unruptured coating was observed.

The coated tubing was then subjected to 10 cycles of wetting, lubricity testing, and drying. It was found that the lubricity did not change even after 10 wetting and drying cycles.

The lubricity was further tested by determining the coefficient of friction using a procedure based on ASTM D-1894. In this test, five uncoated pieces of the thermoplastic tubing were affixed parallel to a stationary platform. The platform with the pieces attached was immersed in 38°C water. A sled (200 gram stainless steel wrapped in latex rubber) was attached to a load cell and transfer mechanism. The sled was placed on the samples and pulled across the surface of the tubing at a rate of six inches per minute. The force (both static and kinetic) required to pull the sled was recorded. The control tubings were replaced with the coated thermoplastic tubings, and the test was rerun. Once again, the force (both static and kinetic) required to pull the sled was recorded. The coated tubes showed a consistent decrease in friction of approximately 50%.

### e. Formation of the Balloon

Balloons were then formed as follows. The coated polyurethane tubings were placed in a mold which has a cavity with dimensions equivalent to the desired size of the balloon to be formed. The non-sealed end of the coated tubings were attached to a nitrogen source. Clamps were times while applying firm finger pressure. After this, the coating still exhibited a continuous red color along the balloon, indicating that it did not wear off. Further examination for lubricity showed that the lubricity of the section of balloon given the above abrasion test was the same as a similar section that was not rubbed.

The integrity of the coating after the abrasion test was further verified by allowing the coated tubing to dry and reexamining the coated surface under a 50x optical microscope. A continuous, unruptured coating was observed.

### Example 2

A tubing measuring 0.017 in. in inner diameter and having an outer diameter of 0.0315 in. was coated and formed into a balloon using the procedure of Example 1. When inflated the balloon measured 0.100 in. in diameter along a central cylindrical section 0.740 in. long. There were conical sections at each end, tapering at one end from 0.100 in. in diameter to 0.034 in. along a length of approximately 0.165 in. at one end and tapering at the other end from 0.100 in. in diameter to 0.040 in. along a length of approximately 0.165 in. at the other end. The wall thickness of the balloon in the center cylindrical portion was about 0.001 in.

In the process of forming the balloon, the coated surface of the tubing underwent a radial expansion of 217% as well as an indeterminate amount of axial expansion.

The surface of the balloon contained a continuous coating of a crosslinked polyurethane poly(ethylene oxide) complex. When viewed under 30 X optical microscope, a smooth coating was observed, free from fissuring, crazing or other sign of coating rupture. The coating continuity was further disclosed by staining the coating, by immersing the coated balloon in 0.5% aqueous Congo Red solution for 20 seconds, followed by rinsing in tap water to remove excess Congo Red. A continuous red film was observed, corresponding to the coating, which absorbs the dye.

The wet coating on the balloon exhibited a high degree of lubricity as determined by rubbing the coated balloon between the fingers while applying a firm pressure, and comparing the base of movement to that observed when performing the same test on a similar wet balloon which did not contain a coating. The uncoated balloon, even when wet, offered significantly more resistance to movement than the coated balloon.

The durability of the coating was determined by placing a coated, stained balloon under a stream of running tap water and rubbing the coating between the finger and thumb for a total of 20 times while applying firm finger pressure After this, the coating still exhibited a continuous red color along the balloon, indicating that it did not wear off. Further examination for lubricity showed that the lubricity of the section of balloon given the above abrasion test was the same as a similar section that was not rubbed.

The integrity of the coating after abrasion test was further verified by allowing the balloon to dry, and reexamining the coated surface under a 30 X optical microscope. A continuous, unruptured coating was observed.

The coated balloon was then subjected to 10 cycles of wetting, lubricity testing, and drying. It was found that the lubricity observed did not change even after 10 wetting and drying cycles.

### Example 3

A coating formulation was prepared by weighing the following components into a disposable beaker:
1) 3.61 grams of CB-60 (the isocyanate described in Example 1);
2) 2.78 grams of a saturated polyester polyol (Multron R-12A, Miles Corporation) which typically has a hydroxyl content of 5%, equivalent weight of 337, specific gravity @ 20°C of 1.13, viscosity @ 20°C of 17,700 mPa's, and flash point (Pensky-Marten closed up) of 320°F (160°C);
3) 150 grams of a 3.3 wt. % methylene chloride solution of Polyox WSR N-750 (the poly(ethylene oxide) described in Example 1); and
4) 283 grams of methylene chloride. The components were thoroughly mixed with a spatula to produce a uniform coating solution having total solids content of 2.25 wt. %.

The citing solution was then transferred to an 18 inch long glass tube having a 3/4 in. outer diameter which was plugged at one end. The glass tube was used as a dipping vessel. Twelve inch lengths of polyethylene terephthalate tubing measuring 0.050 in. by 1.0 in. and having an outer diameter of 0.082 in. were plugged at the bottom end by insertion of a short length of 0.050 in. Teflon rod. The tubings were then dipped in the coating solution to a depth of 9.5 in. during 38 seconds. The tubings were then air dried at ambient conditions for a period of between 15 minutes and 120 minutes and then baked 8 hours at 50°C to effect cure of the coating. Continuous coatings consisting of a crosslinked polyurethane complexed or associated with poly(ethylene oxide) were formed on the tubings.

The tubings were then formed into a dilatation balloon using the stretching, heating, and blow molding methods disclosed in U.S. Re. 32,983 (to S.B. Levy). The tubings were heated at 190°F and then simultaneously pressurized and stretched longitudinally and radially to form the balloons. The balloons were then heated for about 30-40 seconds at about 300°F to crystallize the PET. The final balloons had a central cylindrical segment 1.10 in. long having an outer diameter of approximately 0.320 in., with a conical section at each end. One conical section tapered from 0.320 in. to 0.085 in. along a length of 0.75 in. The other conical section tapered from 0.320 in. to 0.100 in. along a length of 0.75 in. The wall thickness of the coated balloons in the central cylindrical portion was about 0.0015 in. In the process of forming the balloon, the coated surface of the tubing underwent a radial expansion of 290% as well as an indeterminate amount of axial expansion.

There was a continuous coating on the surface of the balloon. The coated surface was viewed under a 30 X microscope and it was free from fissuring, crazing or other signs of coating rupture. The coated balloons were tested for lubricity using the procedure of Example 2 and found to be very slippery when compared to an uncoated balloon having the same dimensions formed by the same process. The coated balloons were then subjected to durability testing for coating integrity after lubricity testing. The ability of the coatings to withstand 10 cycles of wetting, lubricity testing, and drying was also tested. The results were the same as those reported in Example 2.

### Example 4

A coating formulation was prepared by weighing the following components into a disposable beaker:
1) 1.82 grams of CB-60 (the isocyante described in Example 1);
2) 3.86 grams of the polyester resin Multron R-18 (the polyester/polyol described in Example 1);
3) 150 grams of 3.3 wt. % methylene chloride solution of Polyox WSR N-750 (the poly(ethylene oxide) described in Example 1); and
4) 284 grams of methylene chloride.
The components were mixed thoroughly with a spatula to produce a uniform coating solution having a total solids of 2.25 wt. %.

The coating was applied to the polyethylene terephthalate tubing described in Example 3 using the same equipment and dip process. The coated tubing was air dried and cured using the conditions described in Example 3. The coating consisted of a crosslinked polyurethane complexed or associated with poly(ethylene oxide).

The tubing was then formed into a dilatation balloon using the process described in Example 3. The balloon had the same dimensions.

A continuous coating was observed on the balloon surface. The coating became slippery when wetted with water. The coating exhibited durability, integrity after lubricity testing, and retained its lubricity after 10 cycles of wetting, lubricity testing, and drying as described in Example 2.

### Example 5

A coating formulation was prepared by weighing the following components into a disposable beaker:
1) 115.7 grams of a 5 wt. % solution in methylene chloride of a thermoplastic polyesterurethane (Estane 5703F available from B.F. Goodrich Corp.) which has a Brookfield viscosity of 260 cps. at 15% total solids in methyl ethyl ketone (MEK) and of 1600 cps. at 15% total solids in cyclo hexanone; a Tg of -20°C (DSC/second heat), a weight average molecular weight (M_{w}) of 124,000, and tensile @ break of 4000 psi and elongation at break of 750% at 1.5 Mil in MEK and tensile @ break of 3300 psi and elongation @ break of 760% at 1.5 mil in cyclohexanone.
2) 128.6 grams of a 0.75 wt. % solution in methylene chloride of a poly(ethylene oxide) having a mean molecular weight of 2,000,000 (Polyox WSR N-60K; Union Carbide Corp.); and
3) 55.7 grams of methylene chloride.
The components were thoroughly mixed with a spatula to produce a uniform coating solution having a total solids content of 2.25 wt. %. The coating was a blend of the polyurethane and the high molecular weight poly(ethylene oxide). The weight ratio of the polyurethane to poly(ethylene oxide) was 6:1.

The coating was applied to the polyethylene terephthalate tubing described in Example 2 using the same process. A dilatation balloon was blown using the process described in Example 2. The balloon had the same dimensions.

There was a continuous coating on the surface of the balloon, as indicated by microscopic examination under 30 X power. When wetted with water, the coated balloon exhibited a high degree of initial lubricity. However, the coating lost lubricity rapidly when rubbed with firm finger pressure under running water and became non-slippery after 5 rubbing cycles. This was probably due to leaching of some of the poly(ethylene oxide) into the water. After lubricity testing, the surface of the balloon was microscopically reexamined under 30 X power and found to still have continuous coating. Presumably this was the Estane which was not altered by exposure to water, other than by the loss of some of the admixed poly(ethylene oxide).

### Example 6

This example is a comparative example showing that the coating of Example 1 cannot be used on a pre-formed polyurethane balloon.

The coating solution described in Example 3 was prepared. Instead of coating a length of polyethylene terephthalate tubing as before, the coating was applied in a conventional manner. The distal end of a catheter shaft contained a pre-formed, inflated polyethylene terephthalate balloon having the same dimensions as the balloon described in Example 3. The balloon end of the catheter was dipped into the coating solution to a depth of 9.5 in. during 38 seconds as before. The balloon was air dried at ambient conditions for a period of about an hour and then baked 8 hours at 50°C to effect cure of the coating. The result was an adherent coating which exhibited good lubricity and durability when wetted with water. However, there was significant axial shrinkage in the balloon which caused the catheter shaft to buckle in the region between the two adhesively bonded balloon ends. This rendered the catheter useless as a medical device.

### Example 7

A coating formulation was prepared weighing 4.0 grams of a thermoplastic hydrophilic polyurethane polymer, available as D-6/40 from Tyndale Plains-Hunter, Ltd., Ringoes, NJ into an eight ounce glass bottle. A total of 196 grams of methylene chloride was then weighted into the bottle, which was subsequently capped and rolled overnight to produce a uniform solution containing 2 wt. % of the polymer.

Twelve inch lengths of the polyethylene terephthalate tubing described in Example 3 were dip coated in this solution by immersing them at about 1 inch per second to a depth of about 9 inches, then withdrawing them at a speed of about 0.25 inches per second. The coated tubes were air dried at ambient conditions for a period of 20-30 minutes and then baked at 100°C for twenty minutes. The tubes were then formed into a dilatation balloons as described in Example 3. The resulting dimensions and expansion were as described therein.

The balloons exhibited a continuous coating over their entire surface. When viewed under a 30 X microscope the surface was observed to be free of fissuring, crazing or other sign of coating rupture. The balloon surface was wetted with water and found to be more lubricous than a similar uncoated balloon when rubbed between the fingers. Lubricity was not diminished after twenty cycles of rubbing. The coating was stained in 0.5% aqueous Congo Red solution and it was found to be continuous and hydrophilic.

### Example 8

A non-hydrophilic protective slip-coating formulation was prepared by weighing 5.0 grams of a polyfunctional aziridine crosslinker (available as KM10-1703 from Stahl Incorporated, Peabody, MA) into an 8 ounce glass bottle. A total of 195 grams of a polyurethane emulsion containing a polydimethylsiloxane slip agent (available as UE-41-566 form Stahl Incorporated) was then weighed into the bottle which was subsequently capped and rolled 30 minutes to produce a uniform dispersion.

Twelve inch lengths of the polyethylene terephthalate tubing described in Example 3 were dip coated as described in Example 7. The tubings were allowed to air dry at ambient conditions for a period of at least 5 minutes. Then the tubings were baked at 51°C for 20 minutes to crosslink the polyurethane. The tubings were then formed into dilatation balloons as described in Example 3. The resulting dimensions and expansion were as described therein.

The balloons exhibited a continuous coating over their entire surface as evidenced by microscopic examination at 30 X. The dry balloon surface, which contained a significant amount of polydimethyl siloxane slip agent, was observed, when rubbed between the fingers, to feel slippery in comparison to a similar uncoated balloon.

Now that the preferred embodiments of the invention have been given in detail, various modifications and improvements thereon will become readily apparent to those skilled in the art. Accordingly, the spirit and scope of the present invention are to be limited only by the appended claims and not by the foregoing specification.

## Claims

1. A process for preparing a coated expanded thermoplastic medical product, which comprises the steps of:
(a) applying to at least a portion of an inner and/or an outer surface of an expandable thermoplastic tubing at least one adherent functional biomedical coating which comprises at least one biomedical functional agent and a carrier;
(b) allowing the coated tubing to dry at a time and temperature sufficient to remove at least part of the carrier; and
(c) stretching and/or expanding the dried coated tubing with an inflating fluid at a pressure sufficient to form the coated expanded medical product.

2. The process of Claim 1, further comprising the step of heating the partially dried coated tubing at a temperature and for a time sufficient to soften the coated tubing prior to stretching and/or expansion.

3. The process of Claim 1, wherein the tubing is selected from the group consisting of polyethylene; polypropylene; polyethylene terephthalate; polybutylene terephthalate; polyvinyl chloride; polyimides; polyetherimides; polyamides; polyamideimides; polycarbonates; ionomers; polyaryl ether ketones; polyurethane; ethylene-vinyl acetate copolymers; ethylene-butylene-styrene block copolymers admixed with low molecular weight styrene and optionally propylene; and polyether polyamide block copolymers and wherein the coating is a lubricious coating.

4. The process of Claim 3, wherein the substrate is polyurethane or polyethylene terephthalate and wherein the coating contains a poly(ethylene oxide), a polyvinyl pyrrolidone, a hydrophilic polyurethane, or a silicone.

5. The process of Claim 4, wherein the coating contains poly(ethylene oxide) a preformed polymer selected from the group consisting of a polyurethane, a polyurea, and a polyurethane-urea or contains polymer precursors selected from the group consisting of polyurethane precursors and polyurea precursors.

6. The process according to Claim 5, wherein the coating comprises a crosslinked polyurethane matrix.

7. The uniformly functionally coated expanded thermoplastic medical product prepared by the process of Claim 1.

8. The uniformly functionally coated expanded thermoplastic medical product prepared by the process of Claim 3.

9. The uniformly functionally coated expanded thermoplastic medical product prepared by the process of Claim 4.

10. The uniformly functionally coated expanded thermoplastic medical product prepared by the process of Claim 5.

11. The uniformly functionally coated expanded thermoplastic medical product prepared by the process of Claim 6.

12. A process for preparing a coated balloon, which comprises the steps of:
(a) applying to at least a portion of an inner and/or an outer surface of an expandable thermoplastic tubing at least one adherent lubricious coating which comprises at least one lubricious agent and a carrier;
(b) allowing the coated tubing to dry at a time and temperature sufficient to remove at least part of the carrier; and
(c) stretching and/or expanding the dried coated tubing with an inflating fluid at a pressure sufficient to form the coated balloon.

13. The process of Claim 12, further comprising the step of heating the partially dried coated tubing at a temperature and for a time sufficient to soften the coated tubing prior to stretching and/or expansion.

14. The process of Claim 12, wherein the tubing is selected from the group consisting of polyethylene; polypropylene; polyethylene terephthalate; polybutylene terephthalate; polyvinyl chloride; polyimides; polyetherimides; polyamides; polyamideimides; polycarbonates; ionomers; polyaryl ether ketones; polyurethane; ethylene-vinyl acetate copolymers; ethylene-butylene-styrene block copolymers admixed with low molecular weight styrene and optionally propylene; and polyether polyamide block copolymers and wherein the coating is a lubricious coating.

15. The process of Claim 12, wherein the substrate is a polyurethane or polyethylene terephthalate and wherein the coating is a lubricious coating comprising a hydrophilic polyurethane and an organic solvent.

16. The process of Claim 12, wherein the substrate is a polyurethane or polyethylene terephthalate and wherein the coating is a protective lubricious coating comprising an aqueous dispersion of a urethane, a silicone or siloxane emulsion, and optionally a polyfunctional aziridine as a crosslinking agent.

17. The process of Claim 12, wherein the substrate is a polyurethane or polyethylene terephthalate and coating contains poly(ethylene oxide) and a preformed polymer selected from the group consisting of a polyurethane, a polyurea, and a polyurethane-urea or contains polymer precursors selected from the group consisting of polyurethane precursors and polyurea precursors.

18. The process according to Claim 12, wherein the coating comprises a crosslinked polyurethane matrix.

19. The coated balloon prepared by the process of Claim 18.

20. A process for preparing a lubricious dilatation balloon, which comprises the steps of:
(a) applying to at least a portion of an outer surface of a thermoplastic tubing at least one adherent lubricious coating which comprises (i) a carrier, (ii) a poly(ethylene oxide) dissolved, dispersed, or emulsified in the carrier, and (iii) a polymer selected from the group consisting of a polyurethane, a polyurea, and a polyurethane-urea or polymer precursors selected from the group consisting of polyurethane precursors and polyurea precursors, the polymer or polymer precursors being dissolved, dispersed, or emulsified in the carrier;
(b) allowing the coated tubing to dry at a time and temperature sufficient to remove at least part of the carrier; and
(c) expanding the dried coated tubing with an inflating fluid in order to increase the diameter and length of the tubing while simultaneously orienting the tubing and forming the lubricious dilatation balloon.

21. The process of Claim 20, wherein the orienting step includes heating the coated tubing to a temperature within the range extending from the second order transition temperature to the first order transition temperature of the thermoplastic polymer.

22. The process of Claim 21, further comprising the step of drawing the oriented section to provide a drawn oriented section, followed by expanding the drawn oriented section to a modified profile and size, further followed by heat setting the modified profile and size to form the coated balloon.

23. The process of Claim 20, wherein the tubing is selected from group consisting of polyethylene; polypropylene; polyethylene terephthalate; polybutylene terephthalate; polyvinyl chloride; polyimides; polyetherimides; polyamides; polyamideimides; polycarbonates; ionomers; polyaryl ether ketones; polyurethane; ethylene-vinyl acetate copolymers; ethylene-butylene-styrene block copolymers admixed with low molecular weight styrene and optionally propylene; and polyether polyamide block copolymers.

24. The process of Claim 23, wherein the tubing is polyethylene terephthalate.

25. The process of Claim 23, wherein the tubing is polyurethane.

26. The process of Claim 23, wherein the tubing is a segmented polyurethane.

27. The process of Claim 23, wherein the segmented polyurethane contains at least 45 wt.% of soft segments with the balance being hard segments.

28. The process of Claim 23, wherein the carrier is selected from the group consisting of water, methylene chloride, methylene bromide, trichloroethylene, ethylene dichloride, N-methylpyrrolidone, pyridine, and mixtures thereof.

29. The process of Claim 23, wherein the polymer is polyurethane or polyurethane-urea which is prepared from a diisocyanate, a polyether glycol, and a chain extender.

30. The process of Claim 29, wherein the diisocyanate is methylene diisocyanate, the polyether glycol is polyethylene glycol, and the chain extender is selected from the group consisting of water, a low molecular weight diol, a diamine or an amino alcohol of up to carbon atoms, and mixtures thereof.

31. The process of Claim 23, wherein the coating is an intimate physical mixture of about 50-98% by weight of a polyurethane and about 2-50% by weight of a poly(ethylene oxide) having a molecular weight of at least 10,000 in a carrier.

32. The process of Claim 31, wherein the polyurethane is a aliphatic polyurethane and the carrier is water.

33. The process of Claim 23, wherein the coating is a crosslinked polyurethane matrix associated or complexed with a poly(ethylene oxide).

34. The process of Claim 33, wherein the polyurethane matrix is prepared by coating the tubing with a solution consisting essentially of an isocyanate, a polyol, and the polyethylene oxide dissolved in a suitable solvent.

35. The process of Claim 32, wherein the isocyanate is selected from the group consisting of 2,4- and 2,6-toluene diisocyante and prepolymers thereof, hexamethylene diisocyante and prepolymers thereof, diphenylmethane 4,4'-diisocyante and prepolymers thereof, bis(4-isocyanatocyclohexyl) methane and prepolymers thereof, and isophorone diisocyante and prepolymers thereof, wherein the polyol is selected from the group consisting of polyol selected from the group of castor oil and castor oil derivatives, saturated polyester polyols, polyether polyols, unsaturated polyester polyols, polyacrylate polyols; wherein the poly(ethylene oxide) has a molecular weight of about 10,000-5,000,000 and wherein the solvent is selected from the group consisting of solvents methylene chloride, dibromomethane, dichloroethylene, trichloroethylene, pyridine, n-methyle pyrrolidone, dioxolane, acetonitrile, and blends of the above.

36. The lubricious dilatation balloon prepared by the process of Claim 20.

37. The lubricious dilatation balloon produced by the process of Claim 36, wherein the coating is an abrasion resistant, hydrophilic, lubricous coating which is a continuous film of a crosslinked substantially polyurethane matrix complexed or associated with a poly(ethylene oxide).

38. The lubricious dilatation balloon of Claim 37, wherein the crosslinked polyurethane matrix is formed in situ by the reaction of a polyester polyol and toluene diisocyanate in the presence of the polyethylene oxide, with the polyethylene oxide having a molecular weight of above about 50,000.
